# EUROPEAN PATENT SPECIFICATION

(11) **EP 1 414 481 B1**
(45) Date of publication and mention of the grant of the patent: **11.01.2006**
(21) Application number: 02767276.5
(22) Date of filing: 30.07.2002
(51) Int. Cl.: A61K 38/09, A61P 37/06, A61P 25/28

(54) **USE OF CETRORELIX FOR THE TREATMENT OF JACOB-CREUTZFELDT-DISEASE**
VERWENDUNG VON CETRORELIX ZUR BEHANDLUNG VOM JAKOB-CREUTZFELDT-SYNDROM
UTILISATION DU CÉTRORÉLIX POUR LE TRAITEMENT DE LA MALADIE DE CREUTZFELDT-JAKOB

(30) Priority: 31.07.2001 DE 10137174
(43) Date of publication of application: 06.05.2004
(73) Proprietor: Zentaris GmbH, 60314 Frankfurt/Main (DE)
(72) Inventor: ENGEL, Jürgen, 63755 Alzenau (DE); PEUKERT, Manfred, 61440 Oberursel (DE)
(86) International application number: PCT/EP2002/008459
(87) International publication number: WO 2003/011314

(56) References cited:
- WO-A-02/30257
- WO-A-02/30351
- WO-A-91/04479
- WO-A-96/03138
- WO-A-96/04927
- WO-A-96/22786
- WO-A-98/10781
- WO-A-99/26630
- WO-A-02/102401
- WO-A-03/053219
- US-A- 5 663 145
- US-A- 5 843 902
- US-A- 5 985 834
- REISSMANN T ET AL: "The LHRH antagonist Cetrorelix: A review." HUMAN REPRODUCTION UPDATE, vol. 6, no. 4, July 2000 (2000-07), pages 322-331, XP001097713 ISSN: 1355-4786
- FOLDESY R G ET AL: "TREATMENT WITH A NEW LHRH ANTAGONIST ORF-21243 IS AS EFFECTIVE AS COMPLETE CASTRATION IN THE DUNNING PROSTATIC TUMOR MODEL" BIOLOGY OF REPRODUCTION, vol. 34, no. SUPPL. 1, 1986, page 122 XP008020204 ISSN: 0006-3363
- DEBRUYNE F ET AL: "ABARELIX DEPOT, A PURE GNRH ANTAGONIST, COMPARED TO LHRH AGONIST INPATIENTS WITH PROSTATE CANCER" EUROPEAN UROLOGY, S. KARGER AG., BASEL, CH, vol. 37, no. SUPPL 2, 2000, page 128 XP001024927 ISSN: 0302-2838
- ERB KATHARINA ET AL: "Pituitary and gonadal endocrine effects and pharmacokinetics of the novel luteinizing hormone-releasing hormone antagonist teverelix in healthy men: A first-dose-in-humans study." CLINICAL PHARMACOLOGY & THERAPEUTICS, vol. 67, no. 6, June 2000 (2000-06), pages 660-669, XP008020354 ISSN: 0009-9236
- DATABASE WPI Week 200219, 30 October 2001 (2001-10-30) Derwent Publications Ltd., London, GB; AN 2002-011076 XP002249847 FURUYA S., SUZUKI N.: "Preventives/remedies for Alzheimer's disease" & WO 01 78780 A (TAKEDA CHEM IND LTD), 30 October 2001 (2001-10-30)

## Description

### Technical field

The invention relates to the use of the LHRH-antagonist cetrorelix for the manufacture of a medicament for the treatment of Jacob-Creuzfeldt-disease.

### Prior art

In a patent by R.L. Boyd (WO 200062657, AU 200037977) the author claims that disrupting the sex steroid signalling by application of an LHRH-agonist will result in a modification of the T-cell population in subjects with a depressed or abnormal T-cell population. This treatment will have the undesired side-effect of castration of the subject, but the author claims that this castration will be reversible upon cessation of treatment.

This side-effect is highly undesirable as it will result in loss or reduction of libido, sexual desire and sexual potency. In men and pre-menopausal women the treatment would also result in the typical symptoms of lowering the sex hormone-level below castration level, e.g. hot flushes, women will additionally be at risk to lose bone minerals, potentially limiting the duration of treatment.

These unwanted effects can be limited by using an LHRH-antagonist in a dose that will not result in castration but will still have the desired effect on the immune system.

### Description of the invention

The object has now been achieved in that cetrorelix, an LHRH-antagonist, is used for the production of a medicament for treating Jacob-Creuzfeldt-disease.

Expediently, the medicament can be administered in the following ratio:
Total dose from 5 mg to 120 mg cetrorelix, divided in a period of 1 to 8 weeks and according to needs with repeat of the therapy every 3 to 4 months.

It has been found a preferred embodiment of the therapy with the LHRH-antagonist cetrorelix.
- Cetrorelix pamoate in a total dose from 30 mg to 120 mg divided in a period of 1 to 4 weeks and according to needs with repeat of the therapy every 3 to 4 months,
- cetrorelix acetate in a total dose from 5 mg to 80 mg divided in a period of 1 to 8 weeks and according to needs with repeat of the therapy every 3 to 4 months.

## Claims

1. Use of cetrorelix for producing a medicament for the treatment of Jacob-Creuzfeldt-disease.

2. Use according to claim 1, wherein the cetrorelix is to be administered in a dosage that lowers sex hormone levels to a certain extent but not below the castration level.

3. Use according to claim 2, **characterized in that** cetrorelix is to be administered in the following total dose from 5 mg to 120 mg divided in a period of 1 to 8 weeks and according to needs with repeat of the therapy every 3 to 4 months.

4. Use according to claim 2 or 3, **characterized in that** cetrorelix pamoate is to be administered in the following total dose from 30 mg to 120 mg divided in a period of 1 to 4 weeks and according to needs with repeat of the therapy every 3 to 4 months.

5. Use according to claim 2 or 3, **characterized in that** cetrorelix acetate is to be administered in the following total dose from 5 mg to 80 mg divided in a period of 1 to 8 weeks and according to needs with repeat of the therapy every 3 to 4 months.

## Patentansprüche

1. Verwendung von Cetrorelix zur Herstellung eines Medikaments zur Behandlung der Creuzfeldt-Jakob-Krankheit.

2. Verwendung nach Anspruch 1, wobei das Cetrorelix in einer Dosierung zu verabreichen ist, bei der die Sexualhormonspiegel zu einem gewissen Grade gesenkt werden, jedoch nicht unter die Kastrationskonzentration.

3. Verwendung nach Anspruch 2, **dadurch gekennzeichnet, daß** das Cetrorelix in der folgenden Gesamtdosis von 5 mg bis 120 mg, verteilt über einen Zeitraum von 1 bis 8 Wochen und je nach Bedarf unter Wiederholung der Therapie alle 3 bis 4 Monate, zu verabreichen ist.

4. Verwendung nach Anspruch 2 oder 3, **dadurch gekennzeichnet, daß** das Cetrorelixpamoat in der folgenden Gesamtdosis von 30 mg bis 120 mg, verteilt über einen Zeitraum von 1 bis 4 Wochen und je nach Bedarf unter Wiederholung der Therapie alle 3 bis 4 Monate, zu verabreichen ist.

5. Verwendung nach Anspruch 2 oder 3, **dadurch gekennzeichnet, daß** das Cetrorelixacetat in der folgenden Gesamtdosis von 5 mg bis 80 mg, verteilt über einen Zeitraum von 1 bis 8 Wochen und je nach Bedarf unter Wiederholung der Therapie alle 3 bis 4 Monate, zu verabreichen ist.

## Revendications

1. Utilisation du cétrorélix pour fabriquer un médicament destiné au traitement de la maladie de Creutzfeld-Jacob.

2. Utilisation selon la revendication 1,
dans laquelle
le cétrorélix doit être administré dans un dosage qui diminue les niveaux d'hormones sexuelles dans une certaine mesure mais pas au-dessous du niveau de castration.

3. Utilisation selon la revendication 2,
**caractérisée en ce que**
le cétrorélix doit être administré selon la dose totale suivante allant de 5 mg à 120 mg fractionnée sur une période de 1 à 8 semaines et selon les besoins avec un renouvellement de la thérapie tous les 3 à 4 mois.

4. Utilisation selon la revendication 2 ou 3,
**caractérisée en ce que**
le pamoate de cétrorélix doit être administré selon la dose totale suivante allant de 30 mg à 120 mg fractionnée sur une période de 1 à 4 semaines et selon les besoins avec un renouvellement de la thérapie tous les 3 à 4 mois.

5. Utilisation selon la revendication 2 ou 3,
**caractérisée en ce que**
l'acétate de cétrorélix doit être administré selon la dose totale suivante allant de 5 mg à 80 mg fractionnée sur une période de 1 à 8 semaines et selon les besoins avec un renouvellement de la thérapie tous les 3 à 4 mois.
